# EUROPEAN PATENT SPECIFICATION

(11) **EP 1 325 747 B1**
(45) Date of publication and mention of the grant of the patent: **30.07.2008**
(21) Application number: 02425786.7
(22) Date of filing: 19.12.2002
(51) Int. Cl.: A61K 31/555, A61K 31/381, A61K 31/202, A61K 31/198, A61K 31/375, A61K 31/355, A61K 31/51, A61K 31/525, A61K 31/44, A61K 31/714, A23L 1/29, A61P 25/02

(54) **Dietary supplement for neuropaths**
Diätergänzung für neuropatischen Patienten
Supplement diététique pour patients neuropathiques

(30) Priority: 20.12.2001 IT MI20012732
(43) Date of publication of application: 09.07.2003
(73) Proprietor: Health Pharma S.r.l, 20025 Legnano MI (IT)
(72) Inventor: Lucchina, Franco, 21052 Busto Arsizio VA (IT); Terruzzi, Franco, 20040 Briosco MI (IT)
(74) Representative: Pizzoli, Pasquale Vincenzo

(56) References cited:
- WO-A-00/37087
- WO-A-98/33494
- WO-A-98/41113
- WO-A-99/61038
- US-A- 5 292 538
- US-A- 5 895 652
- US-A- 5 922 704
- US-A- 5 962 030
- US-A- 5 976 568
- US-A1- 2002 182 196

## Description

The present invention relates to a dietary supplement and particularly to a composition of substances particularly useful to be given to subjects affected by neuropathies.

It is known that in many cases the dietary supplements are formulated so as to result useful to be given to persons prone to particular deficiency situations or in conditions of increased need of specific nutrients.

The object of the present invention is to provide a dietary supplement particularly useful in the diet in subjects affected by neuropathies. This object is achieved according to the present invention with a composition of substances already known in themselves, but combined in a new and opportunely balanced way to be given in the diet of persons having the aforesaid needs.

The features of the dietary supplement are specified in claim 1, other features are specified in the depending claims.

The dietary supplement according to the present invention besides being easily usable, has the basic advantage of providing the consumer with an aggregate of substances each having a well determined activity and being mutually dosed in an optimal way. It comprises in fact well determined and opportunely co-ordinated amounts of two substances having a neuro-protective activity, a substance having anti-inflammatory and antioxidant activity, a substance acting on the saccharo- and lipometabolism, as well as vitamins with antioxidant properties and a vitamin complex acting on neuronal trophism.

The first substance having neuro-protective activity, opportunely selected for the dietary supplement according to the present invention, is lipoic or thioctic acid, a natural substance whose properties are already known and thus it does not require a detailed description. It is present in the composition in amounts comprised between 50 and 600 mg, preferably between 100 and 300 mg, according to the intake dose and/or dispensing form.

The second substance having a neuro-protective activity that is used in the composition according to the present invention is γ-linolenic acid, a polyunsaturated fatty acid whose properties are already known and thus a detailed description is not necessary for it. It is present in the composition in amounts comprised between 50 and 600 mg, preferably between 100 and 300 mg, depending on the intake dose and/or dispensing form.

As a substance having anti-inflammatory and antioxidant activity, selenium is employed, preferably as selenomethionine comprising 40.26% selenium, in amounts comprised between 0.05 and 0.11 mg, preferably between 0.06 mg and 0.1 mg depending on the intake dose and/or dispensing form.

In some embodiments of the dietary supplement according to the present invention, as a substance having saccharo- and lipometabolism regulating activity chromium picolinate is used comprising 12.43% Cr. Also the properties of this substance are already known and thus a detailed description is not necessary. It is present in the composition in amounts comprised between 0.4 and 1.6 mg preferably between 0.4 and 0.8 mg, depending on the intake dose and/or dispensing form.

Chromium picolinate can possibly be replaced by equivalent amounts of another organic compound of chromium.

As antioxidant vitamins in the composition according to the present invention, vitamins C and E are employed. Vitamin C is introduced in the composition preferably in the form of pure crystals in amounts comprised between 60 and 180 mg, preferably between 90 and 120 mg depending on the intake dose and/or dispensing form.

Vitamin E is present in the composition preferably in the form of 50% powder and in amounts comprised between 1.5 and 30 mg, preferably between 10 and 30 mg depending on the intake dose and/or dispensing form.

Moreover, as a substance acting on neuronal trophism, is present in the composition a vitamin complex of group "B", particularly vitamin B1 in the dosage from 0.21 to 2.1 mg, preferably between 1 and 2 mg; vitamin B2 in the dosage from 0.34 to 2.4 mg, preferably between 1 and 2.2 mg, vitamin B6 in the dosage from 0.3 to 3 mg, preferably between 1 and 3 mg, and vitamin B 12 in the dosage from 0.00035 mg to 0.015 mg, preferably between 0.00075 and 0.0015 mg.

Depending on the intake dose and/or dispensing form, the dietary supplement according to the present invention can also comprise excipients, fragrances and other substances having a known activity for the desired purpose. Such substances are for example maltodextrin, microcrystalline cellulose, magnesium stearate, colloidal silica, etc. Even the optimal amounts of such substances are to be selected each time depending on the dosage and on the dispensing way of the dietary supplement according to the present invention.

Even the preparation technique of the composition according to the present invention is chosen in function of the dispensing way and of other practical considerations. This will be more evident to those skilled in the art from the following embodying example of the present invention.

For the realization of the following operative examples commercially available substances have been used . As α-lipoic acid a product provided by Labochim S.r.1. of Milan has been used; as γ-linolenic acid a product provided from Giellepi Chemicals of Milan has been used, which society has also provided chromium and selenium mineral salts, whereas the substances having vitaminic action have been provided by Istituto delle Vitamine Roche.

### EXAMPLE 1

### Soft gelatin capsules

For the preparation of a dietary supplement according to the present invention in the form of soft gelatin capsules , the different components necessary for the production of an industrial lot of about 150.000 capsules wherein the weight content of the gelatin was about 35%, were separately weighed.

In a stainless steel dissolver provided with stirrer holding about 200 liters, were first introduced γ-linolenic acid, soy-bean lecithin, monodiglycerides of fatty acids followed by all other powdery components under slow stirring.

After a period of about 15 minutes an homogeneous suspension was obtained which was used to fill the soft gelatin capsules through a proper and specific plant working in aseptic environment.

The production plant of soft gelatin capsules worked on the solidification principle of animal gelatin in bands of different width and thickness. The formation of capsules and the relevant sealing was simultaneous with the filling of same by means of suitable moulds. The size of the capsule was an oval 12.

The weight per cent composition of each capsule was finally as follows:

| | |
|---|---|
| Gelatin | 34.3 |
| γ-linolenic acid | 25 |
| α-lipoic acid R/S | 30 |
| Vitamin C | 4.50 |
| Monodiglycerides | 2.80 |
| Vitamin E 50% | 1.50 |
| Soy-bean lecithin | 1 |
| Selenomethionine | 0.01 |
| Pantothenic acid | 0.45 |
| Vitamin B2 | 0.15 |
| Vitamin B1 | 0.12 |
| Vitamin B6 | 0.11 |
| Vitamin B12 | 0.00015 |

The thus obtained soft gelatin capsules, after an appropriate maturing period, were packaged in PVC-ALU blisters or in glass bottles.

### EXAMPLE 2

### Soft gelatin capsules

To make the dietary supplement more suitable to be taken by subjects suffering from saccharometabolism disorders, it was prepared acting as in example 1, but with chromium supply in the form of chromium picolinate.

In a stainless steel dissolver provided with stirrer holding about 200 liters, γ-linolenic acid, soy-bean lecithin, monodiglycerides of fatty acids were first introduced and followed by all other powdery components under slow stirring.

After a period of about 15 minutes an homogeneous suspension was obtained which was used to fill the soft gelatin capsules through a proper and specific plant working in aseptic environment.

The production plant of soft gelatin capsules worked on the solidification principle of animal gelatin in bands of different width and thickness. The formation of capsules and the relevant sealing occurred simultaneously with the filling of same by means of suitable moulds. The size of the capsule was an oval 12.

The final composition of each capsule expressed as a per cent weight was as follows:

| | |
|---|---|
| Gelatin | 34.3 |
| γ-linolenic acid | 25 |
| α-lipoic acid R/S | 30 |
| Vitamin C | 4.50 |
| Monodiglycerides | 2.80 |
| Vitamin E 50% | 1.50 |
| Soy-bean lecithin | 1 |
| Selenomethionine | 0.01 |
| Pantothenic acid | 0.45 |
| Vitamin B2 | 0.15 |
| Vitamin B1 | 0.12 |
| Vitamin B6 | 0.11 |
| Vitamin B12 | 0.00015 |
| Chromium picolinate | 0.08 |

The thus obtained soft gelatin capsules, after an appropriate maturing period, were packaged in PVC-ALU blisters or in glass bottles.

### REFERENCE EXAMPLE 3

### Coated swallowable tablets

To prepare a dietary supplement in the form of coated swallowable tablets the different components necessary for the production of an industrial lot of about 200.000 capsules were separately weighed.

In a Viani type mixer there were thoroughly mixed for about 10 minutes all components according to the present invention. The so obtained mixture was compressed with a Ronchi R23 machine equipped with an electronic device for the control of the unitary weight and provided with suitable oval or circular punches.

The so obtained tablets, weighing about 600 mg, were left for about 48 hours on suitable trellises in a dehumidified room.

The coating of the tablet was carried out in a rotating tray which, to avoid the fusion and consequent polymerization of α-lipoic acid, was heated at temperatures not higher than 40°C.

The coating was accomplished through a suspension of hydroxypropylmethylcellulose, dyes, titanium dioxide and suitable excipients. The final weight of the tablet was 630 mg +/- 20 mg.

The final composition of each capsule expressed as a per cent weight was as follows:

| | |
|---|---|
| Microcrystalline cellulose | 36.32 |
| Racemic α-lipoic acid | 47.37 |
| Selenomethionine | 0.01 |
| Pantothenic acid | 0.71 |
| Vitamin B2 | 0.24 |
| Vitamin B1 | 0.19 |
| Vitamin B6 | 0.17 |
| Vitamin C | 7.11 |
| Vitamin E 50 | 2.37 |
| Hydroxypropylmethylcellulose | 3.16 |
| Magnesium stearate | 0.79 |
| Titanium dioxide | 1.58 |

### REFERENCE EXAMPLE 4

### Coated swallowable tablets

There was followed the same procedure as in example 3, but with chromium supply in the form of chromium picolinate in order to make the dietary supplement more suitable to be taken by subjects suffering from saccharometabolism disorders.

The final composition of the capsule in % of its weight was as follows:

| | |
|---|---|
| Microcrystalline cellulose | 36.32 |
| Racemic α-lipoic acid | 47.37 |
| Selenomethionine | 0.01 |
| Pantothenic acid | 0.71 |
| Vitamin B2 | 0.24 |
| Vitamin B1 | 0.19 |
| Vitamin B6 | 0.17 |
| Vitamin C | 7.10 |
| Vitamin E 50 | 2.37 |
| Chromium picolinate | 0.13 |
| Hydroxypropylmethylcellulose | 3.15 |
| Magnesium stearate | 0.79 |
| Titanium dioxide | 1.58 |

### REFERENCE EXAMPLE 5

There was followed the same procedure as in Example 2 with the difference that 25% of γ-linolenic acid was replaced by phospholipids of soy-bean lecithin in the amount of 10% with respect to the weight of the capsule.

## Claims

1. A dietary supplement for neuropaths comprising α-lipoic acid, γ-linolenic acid, selenium, as well as vitamins having antioxidant activity and vitamins of complex B having neurotrophic activity, wherein α-lipoic acid is comprised in amounts between 50 and 600 mg, γ-linolenic acid comprised in amounts between 50 and 600 mg.

2. A dietary supplement according to claim 1, **characterized in that** vitamin C and vitamin E are contained as antioxidant vitamins.

3. A dietary supplement according to claim 2, **characterized in that** vitamin C is in the form of pure crystals in amounts between 60 and 180 mg and vitamin E is in the form of 50% powder and in amounts between 1,5 and 30 mg.

4. A dietary supplement according to claim 3, **characterized in that** vitamin C is comprised in amounts between 90 and 120 mg and vitamin E is comprised in amounts between 10 and 30 mg.

5. A dietary supplement according to claim 4, **characterized in that** it comprises vitamin B1 in amounts between 0,21 and 2,1 mg, vitamin B2 in amounts between 0,34 and 2,4 mg, vitamin B6 in amounts between 0,3 and 3 mg, and vitamin B12 in amounts between 0,00035 and 0,015 mg.

6. A dietary supplement according to claim 5, **characterized in that** vitamin B 1 is comprised in amounts between 1 and 2 mg, vitamin B2 is comprised in amounts between 1 and 2,2 mg, vitamin B6 is comprised in amounts between 1 and 3 mg, and vitamin B12 is comprised in amounts between 0,00075 and 0,015 mg.

7. Use of α-lipoic acid, γ-linolenic acid, selenium as well as vitamins having antioxidant activity and vitamins of complex B having neurotrophic activity, wherein α-lipoic acid is comprised in amounts between 50 and 600 mg and γ-linolenic acid is comprised in amounts between 50 and 600 mg for preparing a dietary supplement for the treatment of neuropathies.

## Patentansprüche

1. Nahrungsergänzungsmittel bzw. diätisches Mittel für Neuropathien, umfassend α-Liponsäure, γ-Linolensäure, Selen, sowie Vitamine mit antioxidierender Wirkung und Vitamine von Komplex B mit neurotrophischer Aktivität, wobei α-Liponsäure in Mengen zwischen 50 und 600 mg umfasst ist, γ-Linolensäure in Mengen zwischen 50 und 600 mg umfasst ist.

2. Nahrungsergänzungsmittel nach Anspruch 1, **dadurch gekennzeichnet, dass** Vitamin C und Vitamin E als antioxidierende Vitamine enthalten sind.

3. Nahrungsergänzungsmittel nach Anspruch 2, **dadurch gekennzeichnet, dass** Vitamin C in der Form von reinen Kristallen in Mengen zwischen 60 und 180 mg und Vitamin E in der Form von 50% Pulver und in Mengen zwischen 1,5 und 30 mg vorliegt.

4. Nahrungsergänzungsmittel nach Anspruch 3, **dadurch gekennzeichnet, dass** Vitamin C in Mengen zwischen 90 und 120 mg umfasst ist und Vitamin E in Mengen zwischen 10 und 30 mg umfasst ist.

5. Nahrungsergänzungsmittel nach Anspruch 4, **dadurch gekennzeichnet, dass** es Vitamin B1 in Mengen zwischen 0,21 und 2,1 mg, Vitamin B2 in Mengen zwischen 0,34 und 2,4 mg, Vitamin B6 in Mengen zwischen 0,3 und 3 mg und Vitamin B12 in Mengen zwischen 0,00035 und 0,015 mg umfasst.

6. Nahrungsergänzungsmittel nach Anspruch 5, **dadurch gekennzeichnet, dass** Vitamin B1 in Mengen zwischen 1 und 2 mg umfasst ist, Vitamin B2 in Mengen zwischen 1 und 2,2 mg umfasst ist, Vitamin B6 in Mengen zwischen 1 und 3 mg umfasst ist und Vitamin B12 in Mengen zwischen 0,00075 und 0,015 mg umfasst ist.

7. Verwendung von α-Liponsäure, γ-Linolensäure, Selen sowie Vitaminen mit antioxidierender Aktivität und Vitaminen von Komplex B mit neurotrophischer Aktivität, wobei α-Liponsäure in Mengen zwischen 50 und 600 mg umfasst ist, γ-Linolensäure in Mengen zwischen 50 und 600 mg umfasst ist, zum Herstellen eines Nahrungsergänzungsmittels für die Behandlung von Neuropathien.

## Revendications

1. Complément diététique pour névropathes comprenant de l'acide α-lipoïque, de l'acide γ-linolénique, du sélénium, ainsi que des vitamines ayant une activité anti-oxydante et des vitamines du complexe B ayant une activité neurotrophique, dans lequel l'acide α-lipoïque est compris en des quantités entre 50 et 600 mg, et l'acide γ-linolénique est compris en des quantités entre 50 et 600 mg.

2. Complément diététique selon la revendication 1, **caractérisé en ce que** la vitamine C et la vitamine E sont contenues à titre de vitamines anti-oxydantes.

3. Complément diététique selon la revendication 2, **caractérisé en ce que** la vitamine C est sous la forme de cristaux purs en des quantités entre 60 et 180 mg et la vitamine E est sous la forme d'une poudre à 50 % et en des quantités entre 1,5 et 30 mg.

4. Complément diététique selon la revendication 3, **caractérisé en ce que** la vitamine C est comprise en des quantités entre 90 et 120 mg et la vitamine E est comprise en des quantités entre 10 et 30 mg.

5. Complément diététique selon la revendication 4, **caractérisé en ce qu'**il comprend de la vitamine B1 en des quantités entre 0,21 et 2,1 mg, de la vitamine B2 en des quantités entre 0,34 et 2,4 mg, de la vitamine B6 en des quantités entre 0,3 et 3 mg, et de la vitamine B12 en des quantités entre 0,00035 et 0,015 mg.

6. Complément diététique selon la revendication 5, **caractérisé en ce que** la vitamine B1 est comprise en des quantités entre 1 et 2 mg, la vitamine B2 est comprise en des quantités entre 1 et 2,2 mg, la vitamine B6 est comprise en des quantités entre 1 et 3 mg, et la vitamine B12 est comprise en des quantités entre 0,00075 et 0,015 mg.

7. Utilisation d'acide α-lipoïque, d'acide γ-linolénique, de sélénium ainsi que de vitamines ayant une activité anti-oxydante et de vitamines du complexe B ayant une activité neurotrophique, dans laquelle l'acide α-lipoïque est compris en des quantités entre 50 et 600 mg et l'acide γ-linolénique est compris en des quantités entre 50 et 600 mg pour préparer un complément diététique pour le traitement de neuropathie.
